(19) Europäisches Patentamt
European Patent Office
Office européen des brevets

(11) Publication number: 0 362 996 B1

(12) EUROPEAN PATENT SPECIFICATION

(45) Date of publication of patent specification: 27.04.94

(51) Int. Cl.5: A61K 31/12, C07C 50/32, C07C 225/30, C07C 69/157, C07C 69/96, C07C 229/60, C07C 217/22, C07C 46/00

(21) Application number: 89308268.5

(22) Date of filing: 15.08.89

# (54) Naphthoquinones for the treatment and prophylaxis of pneumocystis carinii infections.

(30) Priority: 16.08.88 GB 8819477
16.08.88 GB 8819478
16.08.88 GB 8819479
16.08.88 GB 8819480

(43) Date of publication of application:
11.04.90 Bulletin 90/15

(45) Publication of the grant of the patent:
27.04.94 Bulletin 94/17

(84) Designated Contracting States:
AT BE CH DE ES FR GB GR IT LI LU NL SE

(56) References cited:
DE-A- 2 705 599
GB-A- 2 185 395

(73) Proprietor: THE WELLCOME FOUNDATION LIMITED
Unicorn House
160 Euston Road
London NW1 2BP(GB)

(72) Inventor: Latter, Victoria Susan
The Wellcome Research Laboratories
Langley Court
Beckenham Kent, BR3 3BS(GB)
Inventor: Gutteridge, Winston Edward
The Wellcome Research Laboratories
Langley Court
Beckenham Kent, BR3 3BS(GB)
Inventor: Hudson, Alan Thomas
The Wellcome Research Laboratories
Langley Court
Beckenham Kent, BR3 3BS(GB)

(74) Representative: Rollins, Anthony John et al
Group Patents & Agreements
The Wellcome Research Laboratories
Langley Court
Beckenham Kent BR3 3BS (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 362 996 B1

Rank Xerox (UK) Business Services
(3.10/3.09/3.3.3)

## Description

The present invention is concerned with the use of naphthoquinones for the manufacture of a medicament for the treatment and prophylaxis of Pneumocystis carinii infections.

Pneumocystis carinii is a parasite which has a natural habitat in lung tissue. In a host with a normal immune system P.carinii is not considered to be pathogenic. However, when the immune system is defective P.carinii is liable to cause pneumonia. There is a variety of circumstances in which the immune system may be defective or deficient. Thus, for example immune system deficiency is common in immature or premature infants (neonates). It may also result from suppression by certain drugs, which may be deliberate e.g. in certain patients receiving organ transplants, or unavoidable e.g. as a side-effect of cancer chemotherapy. Disordered growth of one or more constituent parts of the immune system, e.g. as in certain forms of cancer, may also result in immunodeficiency.

Immune deficiency may furthermore be caused by viral infections, including human immunodeficiency virus (HIV). It has been reported (Hughes, W.T. (1987) Treatment and Prophylaxis of Pneumocystis carinii pneumonia, Parasitology Today 3(11) 332-335) that at least 60% of patients with acquired immunodeficiency syndrome (AIDS) suffer from Pneumocystis carinii pneumonia.

In this specification the term "immunocomprised host" will be used to describe hosts with a deficient or defective immune system.

Without treatment, Pneumocystis carinii pneumonia is almost always fatal in immunocompromised hosts. The most widely used treatments for this condition are trimethoprim-sulphamethoxazole (cotrimoxazole) and pentamidine. However, both of these treatments have been reported to be only around 50-70% effective in AIDS patients and to produce a much higher than usual incidence of adverse reactions (about 50%) (Wofsy, C.B. Antimicrobial Agents Annual, 1986, Vol 1, p377-400). There is thus a need for new agents, especially for the prophylaxis of P.carinii pneumonia.

A wide range of naphthoquinones is known in the art. Such compounds have been variously described as having antimalarial, anticoccidial and antitheilerial activity. Some compounds have also been described as possessing activity against external parasites. Thus, Fieser et al, J. Amer. Chem. Soc. 1948, 70, 3156-3165 (and references cited therein) describes a large number of 2-substituted-3-hydroxy-1,4-naphthoquinones as having antimalarial activity. A number of these compounds have also been described in U.S. Patent Specification No. 2 553 648. Further classes of 2-substituted-3-hydroxy-1,4-naphthoquinones having activity as antimalarial, anticoccidial and/or antitheilerial agents are described in U.S. Patents Nos. 3 367 830, and 3 347 742, U.K. Patent Specification No. 1553424, and European Patent Specifications Nos. 2 228, 77551 and 77550.

European Patent Application No. 123239 discloses synergistic combinations of anti-protozoal naphthoquinones and 4-pyridinols or alkanoic esters thereof, which are said to be especially useful for the treatment or prophylaxis of malaria.

European Patent No. 123,238 discloses 2-substituted-3-hydroxy-1,4-naphthoquinones of formula (I)

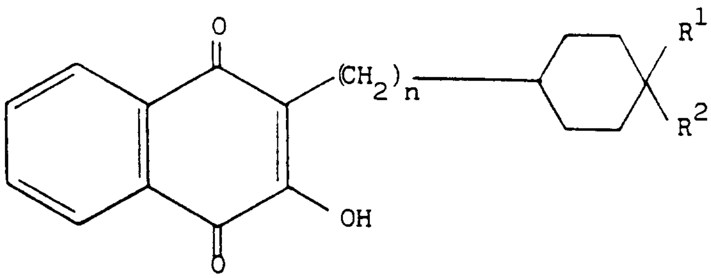

(I)

wherein either $R^1$ is hydrogen and $R^2$ is selected from $C_{1-6}$ alkoxy, aralkoxy,$C_{1-6}$ alkyl-$C_{1-6}$ alkoxy, phenyl substituted by one or two groups selected from halogen and $C_{1-6}$ alkyl, halogen and perhalo-$C_{1-6}$ alkyl or $R^1$ and $R^2$ are both $C_{1-6}$ alkyl or phenyl, and n is zero or 1, and physiologically acceptable salts thereof. Compounds of formula (I) wherein n is zero are said to be active against the human malaria parasite Plasmodium falciparum and also against Eimeria species such as E.tenella and E.acervulina, which are causative organisms of coccidiosis. Compounds of formula (I) where n is 1 are said to be active against protozoa of the genus Theileria, in particular T.annulata and T.parva.

We have now found that a variety of naphthoquinones are active in vivo against Pneumocystis carinii pneumonia infections in rats. Activity has also been demonstrated in an in vitro preparation of P. carinii.

In a first aspect the present invention provides the use of a naphthoquinone for the manufacture of a medicament for the treatment and/or prophylaxis of Pneumocystis carinii infections in mammals (including humans).

Prevention of P.carinii infections is particularly important in an immunocompromised host, as discussed hereinabove. In the case of immunosuppression resulting from HIV infection, prophylaxis may be required by those diagnosed as seropositive for HIV, and those with progressive generalised lymphadenopathy (PGL) or AIDS-related complex (ARC) as well as patients suffering from AIDS.

Naphthoquinones for use according to the present invention are 1,4-naphthoquinones of the general formula (II):

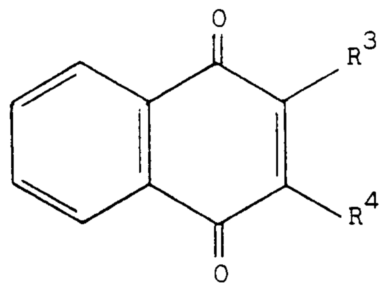

(II)

wherein

$R^3$ is $C_{1-35}$ non-aromatic hydrocarbon residue optionally substituted by one or more substituents, selected from halo, $C_{1-6}$alkoxy, hydroxy, phenyl, phenyl-$C_{1-6}$alkoxy and phenyl-$C_{1-6}$alkyl, each such phenyl group or moiety being optionally substituted by one or more groups selected from $C_{1-6}$alkoxy, $C_{1-6}$alkyl, $C_{1-6}$alkoxy-$C_{1-6}$alkyl, hydroxy, halogen, halo-$C_{1-6}$alkyl, amino, and mono- or di-$C_{1-4}$alkylamino; and

$R^4$ is hydroxy; halogen;

a group $OCOR^5$, wherein $R^5$ is a $C_{1-10}$alkyl group, a $C_{3-10}$ cycloalkyl group, a $C_{1-10}$alkoxy group, or a phenyl or naphthyl group, each such $R^5$ group being optionally substituted e.g. by amino, mono- or di-$C_{1-4}$alkylamino, carboxy or hydroxy;

a group $OR^6$ or $SR^6$, wherein $R^6$ is an optionally substituted $C_{1-10}$alkyl, $C_{3-10}$cycloalkyl, phenyl or naphthyl group as defined for $R^5$; or

a group $NR^7R^8$, wherein $R^7$ and $R^8$ each independently represent hydrogen or $C_{1-4}$alkyl, or the group $NR^7R^8$ represents a 5-7 membered saturated heterocyclic ring, which may optionally contain a further heteroatom selected from nitrogen, oxygen or sulphur;

and physiologically acceptable salts and other physiologically functional derivatives thereof.

A $C_{1-35}$ non-aromatic hydrocarbon residue $R^3$ may be a straight or branched chain $C_{1-14}$ (e.g. $C_{1-8}$)-alkyl or $C_{2-14}$ (e.g. $C_{2-8}$)alkenyl group or a $C_{3-10}$ (e.g. $C_{3-8}$)cycloalkyl group, each of which may optionally carry a $C_{3-10}$ (e.g. $C_{3-6}$)cycloalkyl group, and each of the aforesaid cycloalkyl groups optionally carrying a $C_{1-10}$ e.g. ($C_{1-4}$)alkyl group. The non-aromatic hydrocarbon residue $R^3$ preferably contains from 1 to 20 carbon atoms, e.g. 1 to 14 carbon atoms. Suitable residues $R^3$ include $C_{3-10}$cycloalkyl-$C_{1-8}$-alkyl, $C_{1-10}$alkyl-$C_{3-10}$cycloalkyl, $C_{1-10}$alkyl-$C_{3-10}$cycloalkyl-$C_{1-10}$alkyl and $C_{3-10}$cycloalkyl-$C_{3-10}$cycloalkyl.

Compounds of formula (II) containing an acidic hydroxy or carboxy group, such as compounds wherein $R^4$ is hydroxy, may form salts with bases, and compounds (II) containing a basic amino group may form salts with acids. Suitable base salts include inorganic base salts such as alkali metal (e.g. sodium and potassium) salts and alkaline earth metal (e.g. calcium) salts; organic base salts e.g. phenylethylbenzylamine, dibenzylethylenediamine, ethanolamine and diethanolamine salts; and amino acid salts e.g. lysine and arginine. Suitable acid addition salts include those formed from hydrochloric, hydrobromic, nitric, perchloric, sulphuric, citric, tartaric, phosphoric, lactic, glutamic, oxalic, aspartic, pyruvic, acetic, succinic, fumaric, maleic, oxaloacetic, isethionic, stearic, phthalic, methanesulphonic, p-toluene sulphonic, benzenesulphonic, lactobionic and glucuronic acids.

Without wishing to be bound by theory, it is believed that compounds of formula (II) wherein $R^4$ is a group - $OCOR^5$, $OR^6$, $SR^6$ or $NR^7R^8$ may act as pro-drugs or bioprecursors which are converted in vivo either by the host or the parasite to a compound of formula (II) wherein $R^4$ is hydroxy. Such compounds will be referred to hereinafter as "physiologically functional derivatives". Such compounds may also however possess intrinsic biological activity.

The invention includes within its scope the use of isomers of compounds of formula (II) and mixtures of such isomers. The compounds of formula (II) may exist in a tautomeric form in which the hydroxyl group donates its proton to one of the oxo groups and the use of such tautomeric forms is included within the scope of this invention. However, it is believed that the stable form is that shown in formula (II).

A preferred group of compounds for use according to the invention is that of formula (III):

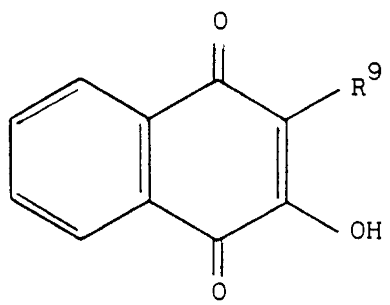

(III)

wherein $R^9$ is

a $C_{1-10}$alkyl group;

a $C_{5-7}$ cycloalkyl group (which may be optionally substituted by a straight or branched chain $C_{1-6}$ alkyl group, a halo-$C_{1-6}$alkyl group, a $C_{1-6}$alkoxy group or a phenyl group, the phenyl group itself being optionally substituted by one or more substituents selected from $C_{1-6}$ alkyl and halogen); or

a $C_{1-10}$alkyl-$C_{5-7}$cycloalkyl group, wherein the cycloalkyl moiety may be optionally substituted as defined for the aforementioned $C_{5-7}$ cycloalkyl group; and

physiologically acceptable salts and other physiologically functional derivatives thereof.

Another group of compounds which may be used according to the present invention is that of formula (IV)

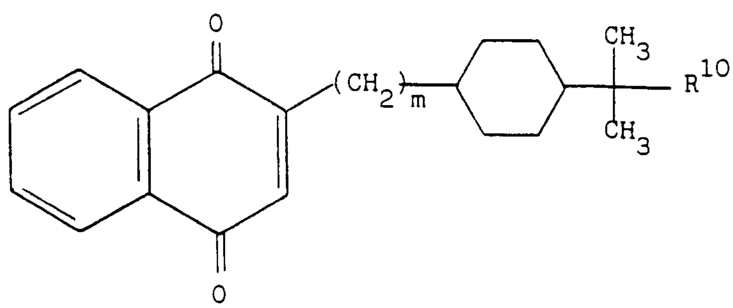

(IV)

wherein $R^{10}$ is an alkyl group of from 1 to 10 carbon atoms and m is O or 1, and physiologically acceptable salts and other physiologically functional derivatives thereof.

In the compounds of formula (IV) $R^{10}$ is suitably a straight-chain $C_{1-4}$ alkyl group, preferably methyl.

A further group of compounds which may be used according to the present invention is that of formula (I) as hereinbefore defined, and physiologically acceptable salts and other physiologically functional derivatives thereof.

Preferred compounds of formula (I) for use according to the present invention include those wherein n is zero, $R^1$ is hydrogen and $R^2$ is phenyl substituted by one or two groups selected from halogen and $C_{1-6}$ alkyl,preferably halogen.

Particularly preferred compounds for use according to the present invention include:

2-(4-t-butylcyclohexyl)-3-hydroxy-1,4-naphthoquinone

2-(4-t-butylcyclohexylmethyl)-3-hydroxy-1,4-naphthoquinone

2-[4-(4-chlorophenyl)cyclohexyl]-3-chloro-1,4-naphthoquinone

and physiologically acceptable salts and physiologically functional derivatives thereof.

An especially preferred compound for use according to the present invention is 2-[4-(4-chlorophenyl)-cyclohexyl]-3-hydroxy-1, 4-naphthoquinone, represented by formula (V):

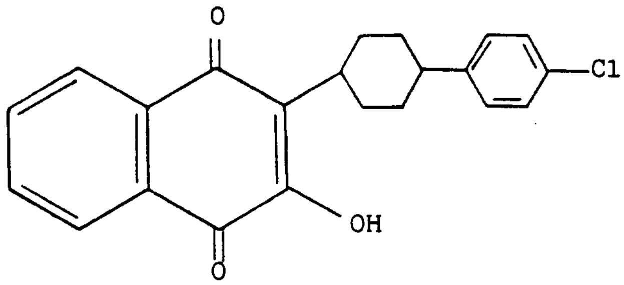

(V)

and physiologically acceptable salts and other physiologically functional derivatives thereof.

Thus, in a preferred aspect the present invention provides the use of the compound of formula (V) and physiologically acceptable salts and other physiologically functional derivatives thereof for the manufacture of a medicament for the treatment and/or prophylaxis of Pneumocystis carinii infections in mammals (including humans).

It will be appreciated that the compounds of formula (I) wherein $R^1$ is hydrogen, and the compounds of formulae (IV) and (V) may exist as the cis or trans isomer, that is to say that the cyclohexyl ring may be cis or trans substituted by the naphthoquinone nucleus and the substituent on the cyclohexyl ring (in the case of formula (V) the chlorophenyl group). Both cis and trans isomers and mixtures thereof in any ratio may be used in accordance with the present invention. In general when the compound is in the form of a mixture of isomers the trans isomer will be present in an amount of about 50% or will be the predominant isomer but the use of mixtures in which the cis isomer predominates is also included within the scope of the invention. The specific ratio of isomers may be varied as required; typical mixtures include those in which the cis/trans isomer ratio is about 1:1,40:60 and 5:95. For use according to the present invention the trans isomer of the compound of formula (V), or a mixture of its cis and trans isomers containing at least 95% e.g. 99% of the trans isomer, is preferred.

Physiologically functional derivatives of the compound of formula (V) include those of formula (VI)

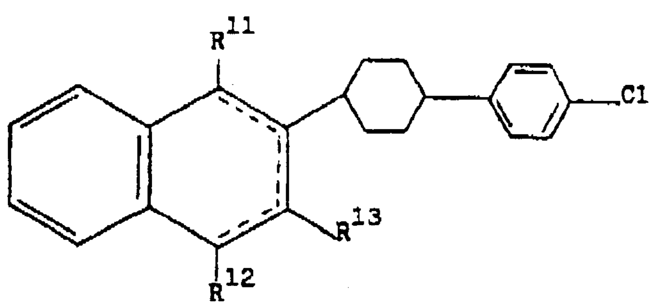

(VI)

wherein

$R^{11}$ and $R^{12}$ each represent =O and the dotted line represents a double bond between the 2 and 3 positions of the quinone ring, in which case $R^{13}$ represents a group $-OCOR^5$; a group $OR^6$ or $SR^6$; or a group $NR^7R^8$, wherein $R^5$, $R^6$, $R^7$ and $R^8$ are as hereinbefore defined.

Further derivatives of the compound (V) which may be used in accordance with the present invention are those of formula (VII)

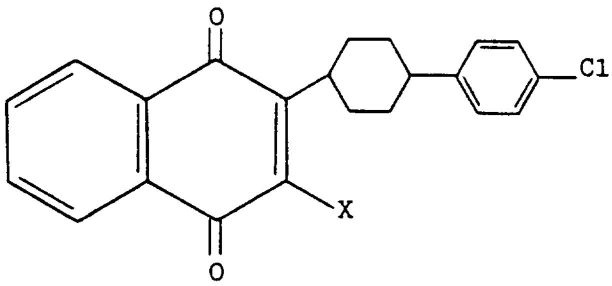

(VII)

wherein X is a halogen atom, e.g. a chlorine, bromine or iodine atom, preferably a chlorine atom.

The compound of formula (VII) wherein X is chlorine has previously been described as an intermediate e.g. in the preparation of the compound of formula (I) but no biological activity has been ascribed to it. In a further aspect therefore the present invention provides a compound of formula (VII) for use as a medicament for the treatment of Pneumocystis carinii infections.

The synthesis of compounds of formulae (I) to (VII) may be effected by methods already known and described in the chemical literature (for example the patent specifications listed hereinbefore) or by analogous methods. In particular novel compounds of formula (VI) may be prepared by the following methods which form a further aspect of this invention:

(a) reaction of a compound of formula (V) or (VII), with a compound serving to introduce the required group $R^{13}$, and where appropriate the groups $R^{11}$ and $R^{12}$;

(b) reaction of a compound of formula (VIII):

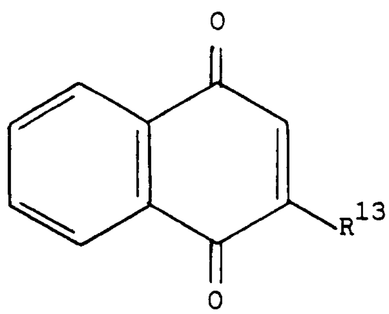

(VIII)

wherein $R^{13}$ is as defined above with a donor compound serving to introduce the 4-(4-chlorophenyl)-cyclohexyl group.

With regard to process (a) compounds (VI) wherein $R^{11}$ and $R^{12}$ represent = 0 and $R^{13}$ represents a group $OCOR^5$ may be prepared by esterification of the compound (V). Esterification may be effected in conventional manner using the appropriate acid $R^5COOH$ or acid derivative e.g. an acid anhydride, acid chloride or an activated ester such as an alkylhaloformate e.g. an alkylchloroformate. To prepare a compound of formula (VI) wherein $R^{11}$, $R^{12}$ and $R^{13}$ each represent a group-$OCOR^4$, the esterification is carried out in the presence of a reducing agent, e.g. zinc.

Compounds of formula (VI) wherein $R^{13}$ is a group $OR^6$ or $SR^6$ may be prepared from a compound (VII) wherein X is a halogen atom. Thus for example the group $OR^6$ may be introduced by reaction with the appropriate alcohol, e.g. methanol or ethanol in the presence of sodium, and the group $SR^6$ may be introduced by reaction with the corresponding thiol, $R^6SH$.

Compounds of formula (VI) wherein $R^{13}$ is $-NR^7R^8$ may be prepared by reduction of the corresponding compound wherein $R^{13}$ is azido, e.g. using lithium aluminium hydride in tetrahydrofuran, followed where necessary and/or desired by alkylation of the resulting amino group. The azido compound may be prepared from a compound of formula (VII) wherein X is halogen, by reaction e.g. with sodium azide.

Compounds of formula (VII) may be prepared for example in an analogous manner to process (b) described below.

With regard to process (b), a suitable donor compound is the corresponding cycloalkane carboxylic acid which may undergo oxidative decarboxylation. For instance persulphate with a catalyst, such as silver ions, is convenient for the purpose, (c.f.Jacobson, N., et al., Annalen, 1972, 763, 135 and Acta Chem. Scand,

1973, 27, 3211). Conveniently ammonium persulphate can be used as the oxidising agent, and the catalyst is silver nitrate. Further details of this process are described in EPA 123238. The compound of formula (VIII) used as starting material may be prepared from the corresponding 3-halo compound using methods analogous to process (a).

Hereinafter naphthoquinones active against P.carinii, including compounds more particularly described by formulae (I) to (VI), and their physiologically acceptable salts and other physiologically functional derivatives will be referred to as the "naphthoquinone". It will be appreciated that the amount of the naphthoquinone required for use in the treatment or prophylaxis of P.carinii will depend inter alia on the activity of the particular compound, the route of administration, the age and weight of the patient and the severity of the condition being treated. In general, a suitable dose for administration to man for the treatment of P.carinii pneumonia may be in the range of 0.1mg to 200mg per kilogram bodyweight per day, for example from 1mg/kg to 100mg/kg, particularly 10 to 50 mg/kg. For administration by inhalation the dose may conveniently be in the range of 0.1 to 20 mg/kg/day, e.g. 0.5 to 10 mg/kg/day. It will be appreciated that for administration to neonates, lower doses may be required.

For use in prophylactic treatment the naphthoquinone may also be given less frequently, e.g. as a single dose on alternate days, once or twice per week or once or twice per month. The dosage for prophylactic treatment will depend inter alia on the activity of the naphthoquinone, the frequency of administration, and, where a depot preparation or controlled release formulation is used the rate of release of the active ingredient. Thus for once-weekly administration a suitable prophylactic dose could be in the range 0.05 to 100 mg/kg,e.g. 0.05 to 50 mg/kg particularly 5 to 50 mg/kg.

For use according to the present invention the naphthoquinone is preferably presented as a pharmaceutical formulation.

Pharmaceutical formulations comprise the naphthoquinone or a physiologically acceptable salt or other physiologically functional derivative thereof together with one or more pharmaceutically acceptable carriers therefor and optionally other therapeutic and/or prophylactic ingredients. The carrier(s) must be acceptable in the sense of being compatible with the other ingredients of the formula and not deleterious to the recipient thereof.

The naphthoquinone may conveniently be presented as a pharmaceutical formulation in unit dosage form. A convenient unit dose formulation contains the naphthoquinone in an amount of from 10 mg to 3g e.g. 10 mg to 1g.

Pharmaceutical formulations include those suitable for oral, topical(including dermal,buccal and sublingual),rectal or parenteral (including subcutaneous, intradermal, intramuscular and intravenous), nasal and pulmonary administration e.g. by inhalation. The formulation may, where appropriate, be conveniently presented in discrete dosage units and may be prepared by any of the methods well known in the art of pharmacy. All methods include the step of bringing into association the naphthoquinone with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules or tablets each containing a predetermined amount of the naphthoquinone. A tablet may be made by compression or moulding, optionally with one or more accessory ingredients. Compressed tablets may be prepared by compressing in a suitable machine the naphthoquinone in a free-flowing form such as a powder or granules optionally mixed with a binder, lubricant, inert diluent, lubricating agent, surface-active agent or dispersing agent. Moulded tablets may be made by moulding an inert liquid diluent. Tablets may be optionally coated and, if uncoated, may optionally be scored. Capsules may be prepared by filling the naphthoquinone, either alone or in admixture with one or more accessory ingredients, into the capsule shells and then sealing them in the usual manner. Cachets are analogous to capsules wherein the naphthoquinone together with any accessory ingredient(s) is sealed in a rice paper envelope. The naphthoquinone compound may also be formulated as dispersible granules, which may for example be suspended in water before administration, or sprinkled on food. The granules may be packaged e.g. in a sachet. Formulations suitable for oral administration wherein the carrier is a liquid may be presented as a solution or a suspension in an aqueous liquid or a non-aqueous liquid, or as an oil-in-water liquid emulsion.

Formulations for oral administration include controlled release dosage forms e.g. tablets wherein the naphthoquinone is formulated in an appropriate release - controlling matrix, or is coated with a suitable release - controlling film. Such formulations may be particularly convenient for prophylactic use.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of the naphthoquinone with the softened or melted carrier(s) followed by chilling and shaping in moulds.

Pharmaceutical formulations suitable for parenteral administration include sterile solutions or suspensions of the naphthoquinone in aqueous or oleaginous vehicles. Injectable preparations may be adapted for bolus injection or continuous infusion. Such preparations are conveniently presented in unit dose or multi-dose containers which are sealed after introduction of the formulation until required for use. Alternatively, the naphthoquinone may be in powder form which is constituted with a suitable vehicle, such as sterile, pyrogen-free water, before use.

The naphthoquinone may also be formulated as a long-acting depot preparation, which may be administered by intramuscular injection or by implantation e.g. subcutaneously or intramuscularly.Depot preparations may include, for example, suitable polymeric or hydrophobic materials, or ion-exchange resins. Such long-acting formulations are particularly convenient for prophylactic use.

Formulations suitable for pulmonary administration via the buccal cavity are presented such that particles containing the naphthoquinone and desirably having a diameter in the range 0.5 to 7 microns are delivered into the bronchial tree of the recipient. Such formulations may be in the form of finely comminuted powders which may conveniently be presented in a pierceable capsule, for example of gelatin, for use in an inhalation device, or as a self-propelling formulation (also referred to as an aerosol formulation) comprising the naphthoquinone, a suitable liquid or gaseous propellant and optionally other ingredients such as a surfactant and/or a solid diluent. Suitable liquid propellants include propane and the chlorofluorocarbons, and suitable gaseous propellants include carbon dioxide. Suitable surfactants include sorbitan trioleate (which is available for example under the trade name "Arlacel 85"), Polysorbate 20 and oleic acid. Self-propelling formulations may also be employed wherein the active ingredient is dispensed in the form of droplets of solution or supension. The self-propelling formulation typically contains from 0.05 to 20mg/ml e.g. 0.1 to 5mg/ml of the active ingredient.

Such self-propelling formulations are analogous to those known in the art and may be prepared by established procedures. Suitably they are presented in a container provided with either a manually-operable or automatically functioning valve having the desired spray characteristics; advantageously the valve is of a metered type delivering a fixed volume, for example, 25 to 100 microlitres, upon each operation thereof.

As a further possibility the naphthoquinone may be in the form of a solution or suspension for use in an atomiser or nebuliser whereby an accelerated airstream or ultrasonic agitation is employed to produce a fine droplet mist for inhalation. Such solutions or suspensions may comprise, in addition to the naphthoquinone and solvent(s), optional ingredients such as surfactants. Suitable surfactants include those described above for self-propelling formulations. The solution or suspension typically contains from 0.05 to 20mg/ml e.g. 0.1 to 5mg/ml of the naphthoquinone. When a suspension of the naphthoquinone is employed, this compound is preferably in finely divided form, e.g. in micronised form.

Formulations suitable for nasal administration include presentations generally similar to those described above for pulmonary administration. When dispensed such formulations should desirably have a particle diameter in the range 10 to 200 microns to enable retention in the nasal cavity; this may be achieved by, as appropriate, use of a powder of a suitable particle size or choice of an appropriate valve. Other suitable formulations include coarse powders having a particle diameter in the range 20 to 500 microns, for administration by rapid inhalation through the nasal passage from a container held close up to the nose, and nasal drops comprising 0.2 to 5% w/v of the naphthoquinone in aqueous or oily solution or suspension.

It should be understood that in addition to the aforementioned carrier ingredients the pharmaceutical formulations for the various routes of administration described above may include, as appropriate one or more additional carrier ingredients such as diluents, buffers, flavouring agents, binders, surface active agents, thickeners, lubricants, preservatives (including anti-oxidants) and the like, and substances included for the purpose of rendering the formulation isotonic with the blood of the intended recipient.

None of the references listed hereinabove contains an invitation to administer the compounds of formula (II) by the nasal or pulmonary route or any suggestion that the said compounds, if administered in such a manner, would be effective in the treatment of the conditions therein taught; the said disclosures likewise contain no description of any formulation suitable for administration by the nasal or pulmonary route.

Pharmaceutical formulations of the compounds of formula (II) adapted for administration by the nasal or pulmonary route are believed to represent novel formulations and thus form a further feature of the present invention.

Novel compounds of formula (VI) may also be formulated in the manner described above for use in the treatment and/or prophylaxis of malaria and such formulations form a further aspect of the present invention.

The above naphthoquinones may also be used in accordance with the present invention in combination or concurrently with other therapeutic agents, for example agents used in the treatment of immunocompromised patients, including anticancer agents such as interferons e.g. alpha-interferons; antiviral agents

such as azidothymidine (AZT,zidovudine), immunostimulants and immunodulators. The naphthoquinone may also be administered in combination with a 4-pyridinol compound, as described in EPA 123,239 e.g. 3,5-dichloro-2,6-dimethylpyridinol (meticlorpindol).

The following non-limiting examples illustrate inter alia the following aspects of the present invention:

the use of naphthoquinones for the manufacture of a medicament for the treatment and prophylaxis of P.carinii infections and

novel pharmaceutical formulations.

Example 1 - Preparation of compound (V)

2-[trans-4-(4-Chlorophenyl)cyclohexyl]-3-hydroxy-1,4-naphthoquinone

a) 4-(4-Chlorophenyl)cyclohexane-1-carboxylic Acid

Acetyl chloride (30g) and finely powdered aluminium chloride (60g) were stirred together in carbon disulphide (120 ml) and then cooled to -50 °C, in a $CO_2$/oxitol bath. Cyclohexene (30 g), previously cooled to -50 °C, was added dropwise during 10 minutes while maintaining the temperature of the reaction mixture at below -20 °C. The mixture was stirred at -50 °C for a further 60 minutes and the solvent then decanted to leave a gummy orange complex. A little chlorobenzene was added as the material warmed to ambient temperature; the remainder of the chlorobenzene (total 300 ml) was then added, the so-obtained solution heated at 40 °C for 3 hours with stirring, poured onto a mixture of ice and concentrated hydrochloric acid and the organic layer separated, washed with 2M hydrochloric acid, 2M sodium hydroxide and water, dried over anhydrous sodium sulphate and evaporated to dress. The product was distilled in vacuo, the fraction boiling at 140-154 °C (0.1 mm Hg) collected, diluted with an equal volume of petroleum ether (40-60), cooled to -6 °C and a continuous stream of nitrogen gas bubbled through, and the separated colourless solid recovered.

Bromine (2.8ml) was added to a solution of sodium hydroxide (6.2g) in water (42 ml) at 0 °C. The above-obtained substituted hexahydroacetophenone (3.1g) was dissolved in dioxan (15 ml) and the cold hypobromite solution then added, keeping the reaction mixture at below 20 °C. The reaction mixture was stirred at ambient temperature for 6 hours then allowed to stand overnight. Sodium metabisulphite was added to destroy excess hypobromite, the mixture cooled and then acidified to give a colourless solid. The solid was filtered off, washed with water, dried and recrystallised from ethanol to give 4-(4-chlorophenyl)cyclohexane-1-carboxylic acid, m.p. 254-256 °C.

b) 2-[4-(4-chlorophenyl)cyclohexyl]-3-chloro-1,4-naphthoquinone

A mixture of 2-chloro-1,4-naphthoquinone (3.95g, 0.02 mol), 4-(4-chlorophenyl)cyclohexane-1-carboxylic acid (4.9g, 0.02 mol) and powdered silver nitrate (1.05g, 0.0062 mol) was heated to reflux with vigorous stirring in 40 ml of acetonitrile. A solution of ammonium persulphate (12.0g, 0.0525 mol) in 50 ml of water was added dropwise over 1 hour. The mixture was refluxed for 3 hours then cooled in ice for 30 mins, after which it was filtered, and the residual sticky solid extracted twice with boiling chloroform to remove inorganic material. The chloroform was removed by evaporation to leave a yellow-brown solid (ca 2.7g). This was dissolved in 40 ml of boiling acetonitrile; a little insoluble material was removed by filtration. On cooling, the title compound separated as yellow crystals, (550 mg) m.p. 172-175 °C.

NMR, $\delta$H($d_6$-DMSO) 8.05 (2H, mult., $\beta$-naphth), 7.85(2H, mult., $\alpha$-naphth), 7.30 (4H, s., PhH), 3.30 (1H, br.t., CH), 2.67 (1H, br.t., CH), 1.2-2.4 (8H, mult., 4x$CH_2$).

c) 2-[4-(4-chlorophenyl)cyclohexyl]-3-hydroxy-1,4-naphthoquinone

The product of stage (b) was suspended in 10 ml of boiling methanol and 0.55g of potassium hydroxide in 5.5 ml of water was added dropwise over 15 mins. The mixture was refluxed until a dark red solution formed, (after ca. 6 hrs) when 2 ml of concentrated hydrochloric acid was cautiously added dropwise. The mixture was cooled and filtered, and the solid residue washed thoroughly with water. The water washings were re-acidified and filtered. The combined solid residues (500 mg) mp 200-209 °, were recrystallised from acetonitrile to give the title product as the trans-isomer (300 mg) m.p. 216-219 °C.

Example 2

2-Methoxy-3-[trans-4-(4-chlorophenyl)cyclohexyl]-1,4-naphthoquinone

Sodium (0.3g, 0.013mol) was dissolved in 20ml of methanol and the compound of Example 1(b) (1.5g, 0.004mol) was added. The mixture was warmed to reflux for 4 hours, then evaporated under reduced pressure. The dark red solid which remained was partitioned between water and chloroform. The chloroform

layer was washed with ice cold dilute sodium hydroxide, followed by water and was then dried and evaporated to give a yellow solid (900mg). This was recrystallised from acetonitrile to give the impure product (800mg) mp117-120°, which was further recrystallised from ethanol to give the title compound - (600mg) mp120-122°.

NMR, δH ($d_6$-DMSO) 8.0 (2H, mult., β-naphth), 7.85 (2H, mult, α-naphth) 7.35 (4H, s, PhH), 4.0 (3H, s, OMe), 3.1 (1H, br.t., CH), 2.6 (1H, br.t., CH), 1.5-2.2 (8H, m, $4xCH_2$).

Example 3

2-Amino-3-[trans-4-(4-chlorophenyl)cyclohexyl]-1,4-naphthoquinone

a) 2-azido-3-[trans-4-(4-chlorophenyl)cyclohexyl]-1,4-naphthoquinone

A solution of sodium azide (0.42g, 0.006mol) in 6ml of water was added to a suspension of the compound of Example 1(b) (1.1g, 0.003mol) in 15ml of ethanol. The mixture was heated to reflux with stirring and then a further 15ml of ethanol and 6ml of water were added. Heating under reflux was continued for 4 hours followed by cooling in a refrigerator for 1 hour. The resulting yellow crystals were filtered off and washed with water and ethanol to give the impure title compound (0.9g) mp130-135°. This material was used in the next stage without further purification.

b) 2-Amino-3-[trans-4-(4-chlorophenyl)cyclohexyl]-1,4-naphthoquinone

The impure product of stage (a) (0.9g) was dissolved in dry tetrahydrofuran (THF) and added dropwise to a suspension of lithium aluminium hydride (2.0g) in THF. The mixture was stirred at room temperature for 1 hour and then 2.0ml of water was added dropwise with caution. A current of air was passed through the mixture for 1 hour and then 0.7g of sodium hydroxide in 6ml of water was added. The mixture was filtered and washed with THF. The filtrate was evaporated to dryness leaving an amorphous orange material which was triturated with SVM whereupon orange crystals formed. These were filtered off, washed well with SVM and dried to yield a first crop of product (200mg) mp210-215°. The reaction was repeated to give a further crop of product (300mg) mp200-210°. The two crops were combined and chromatographed, eluting with chloroform, to give the title compound (350mg) mp212-215°.

NMR, δH ($d_6$-DMSO) 8.0 (2H, mult., naphth) 7.8 (2H, mult., naphth), 7.35 (4H, q, PhH), 6.7 (2H, s, $NH_2$), 2.6-3.0 (2H, mult., CH), 1.5-2.4 (8H, mult., $CH_2$).

Example 4

2-[trans-4-(4-Chlorophenyl)cyclohexyl]-3-(3-dimethylaminopropoxy)-1,4-naphthoquinone hydrochloride

Sodium (0.10g,4.5mmol) was dissolved in 3-dimethylamino-propan-1-ol (1.55g, 5eq) and cooled and the compound of Example 1(b) (1.15g, 3mmol) was added. The mixture was stirred at room temperature for 1 hour, following which acetic acid (1ml) was added and the mixture diluted with toluene (30ml). The solution was washed with water (4 x 20ml) dried ($MgSO_4$) and evaporated in vacuo to a purple semi solid. This was taken up in acetone and a mixture of diethyl ether and hydrochloric acid added until the purple solution turned orange. The solution was evaporated in vacuo to dryness and washed several times with toluene to give a yellow/beige solid, which was recrystallised from methanol/toluene (1:99) to give the title compound as a yellow/green crystalline solid (0.92g) mp191-194°.

NMR, δH ($CDCl_3$) 8.06 (2H, mult., β-naphth), 7.74 (2H, mult., α-naphth), 7.25 (4H, mult., PhH), 4.28 (2H, t., $OCH_2$ ), 3.48 (2H, t., $NCH_2$ ), 2.95 (6H, s., 2xMe), 3.17 (1H, br.t., CH), 2.67 (1H, br.t., CH), 2.54 (2H, mult., $CH_2$) 1.4-2.3 (8H, mult., $4xCH_2$).

Example 5

2-Acetoxy-3-[trans-4-(4-chlorophenyl)cyclohexyl]-1,4-naphthoquinone

The compound of Example 1(c) (1.5g, 0.004mol) was suspended in acetic anhydride (3ml) and a few drops of concentrated sulphuric acid were added with vigorous stirring. A further quantity of acetic anhydride (3ml) was then added, the mixture stirred for 30 minutes and then poured into 15ml of water causing a vigorous reaction. The resulting mixture was cooled in ice and filtered to give pale yellow crystals which were washed with water and dried to give the impure product (1.6g) mp149-152°. This was recrystallised from 100ml of SVM to give the title compound (1.3g) mp158-160°.

NMR, δH ($d_6$-DMSO) 8.1 (2H, m, β-naphth), 7.9 (2H, m, α-naphth), 7.35 (4H, s, PhH), 3.1 (1H, br.t., CH), 2.6 (1H, br.t., CH), 2.5 (3H, s, COMe), 1.5-2.0 (8H, m, $CH_2$).

Example 6

2-Ethoxycarbonyloxy-3-[trans-4-(4-chlorophenyl)cyclohexyl]-1,4 naphthoquinone

The compound of Example 1(c) (1.1g, 0.003mol) and pyridine (0.24g, 0.003mol) was stirred in 5ml of toluene and cooled in a water bath while 4ml of ethyl chloroformate was added dropwise over a period of 15 minutes. The mixture was stirred for a further 30 minutes then poured into a mixture of ethyl acetate and water. The organic layer was separated, dried and evaporated to a yellow solid which was recrystallised from chloroform/petrol to give the impure product (850mg) mp145-149°. This material was dissolved in chloroform, washed several times with ice cold 0.1N sodium hydroxide and then water. The organic layer was dried and evaporated to give product (450mg) mp147-9°. The reaction was repeated on the same scale, the product combined with the aforementioned material and then recrystallised from chloroform/petrol to give the title compound (1.3g) mp153-155°.

NMR, δH ($d_6$-DMSO) 8.1 (2H, m, β-naphth), 7.9 (2H, m, α-naphth), 7.4 (4H, s, PhH), 4.3 (2H, q, $OCH_2$), 3.1 (1H, br.t., CH), 2.6 (1H, br.t., CH), 1.5-2.0 (8H, m, $CH_2$), 1.4 (3H, t, Me).

Example 7

2-[trans-4-(4-Chlorophenyl)cyclohexyl]-3-(4-dimethylaminobenzoyloxy)-1,4-naphthoquinone

The compound of Example 1(c) (2.0g, 5.4mmol) in dry toluene (50ml) containing dry pyridine (0.44g, 1eq) was stirred at around room temperature. 4-Dimethylaminobenzoyl chloride (1g, 1eq) in dry toluene (25ml) was added dropwise over 15 minutes. The mixture was stirred at around room temperature for 1 hour, heated at reflux for 10 hours, left standing for 38 hours, and then refluxed for a further 7 hours. The mixture was then cooled, washed with water, sodium bicarbonate solution and again with water, dried ($MgSO_4$) and evaporated in vacuo to a yellow solid which was recrystallised from ethanol to give the title compound (1.25g) mp117-121° (shrinks above 113°).

NMR, δH ($CDCl_3$) 8.1, 6.75 (4H, mult., β-naphth + α-$Me_2$N-Ph), 7.72 (2H, mult., α-naphth), 7.18 (4H, mult., PhH), 6.72 (2H, d, β-$Me_2$N-Ph), 3.18 (1H, br.t., CH), 3.14 (6H, s., 2xMe), 2.52 (1H, br.t., CH), 1.4-2.2 (8H, mult., 4x$CH_2$).

Example 8

2-[trans-4-(4-Chlorophenyl)cyclohexyl]-1,3,4-triacetoxynaphthalene

The compound of Example 1(c) (1.0g) and zinc dust (1.0g) was stirred at room temperature for 24 hours in acetic anhydride (6ml) with one drop TEA. The reaction mixture was filtered and added to water (50ml) and stirred for one hour. The resulting white precipitate was filtered, washed with water (4 x 20ml) and dried to give the title compound (0.4g) mp177-179°.

NMR, δH ($d_6$-DMSO) 7.87 (2H, mult., β-naphth), 7.63 (2H, mult., α-naphth), 7.35 (4H, s., Ph.H), 2.95 (1H, br.t., CH), 2.64 (1H, br.t., CH), 2.62 (3H, s., OAc), 2.49 (3H, s., OAc), 1.4-2.3 (8H, mult., 4x$CH_2$).

Example 9

The following examples illustrate conventional pharmaceutical formulations which may be employed in accordance with the present invention:-

A. Injectable solution

A solution for intramuscular injection may be prepared by mixing:-

| | |
|---|---|
| Compound of Example 1 | 9.5 parts by weight |
| Dimethyl sulphoxide | 19.0 parts by weight |
| Sorbitan monooleate | 4.5 parts by weight |
| Corn oil | 67.0 parts by weight |
| | 100.0 |

B. Injectable solution

| | |
|---|---|
| Compound of Example 1 | 5 parts by weight |
| N-methyl-pyrollidone | 48.3 parts by weight |
| Tween® 80 | 2 parts by weight |
| Span® 80 | 4.7 parts by weight |
| Miglyol® 812 | 40 parts by weight |
| | 100.0 |

C. Tablet

| | |
|---|---|
| Compound of Example 1 | 25.0 mg |
| Lactose BP | 48.5 mg |
| Microcrystalline Cellulose BP ("Avicel® pH 101") | 10.0 mg |
| Low-substituted Hydroxypropyl; Cellulose BP ("LHPC LH-11") | 10.0 mg |
| Sodium Starch Glycollate BP ("Explotab") | 3.0 mg |
| Povidone BP ("K30") | 3.0 mg |
| Magnesium Stearate BP | 0.5 mg |
| | 100.0 mg |

D. Oral suspension

| | |
|---|---|
| Compound of Example 1 | 50 mg |
| Avicel® RC 591 | 75 mg |
| Sucrose syrup | 3.5 ml |
| Methylhydroxybenzoate | 5 mg |
| Colour | 0.01% w/v |
| Cherry flavour | 0.1 % v/v |
| Tween® 80 | 0.2 % v/v |
| Water | to 5 ml |

E. Injectable suspension

| | |
|---|---|
| Compound of Example 1 | 100 mg |
| Polyvinyl pyrrolidone (PVP) | 170 mg |
| Tween® 80 | 0.2% v/v |
| Methylhydroxybenzoate | 0.1% w/v |
| Water for Injection | to 3 ml |

F. Capsule

| | |
|---|---|
| Compound of Example 1 | 100 mg |
| Starch 1500 | 150 mg |
| Magnesium stearate filled into a hard gelatin capsule | 2.5 mg |

Example 10.

The following examples illustrate novel pharmaceutical formulations according to the present invention.

A. Suspensions for Nebulisation

a)

| | |
|---|---|
| Compound of Example 1, sterile | 1.0 mg |
| Water for Injections | to 10.0 ml |

Disperse the naphthoquinone in the Water for Injections previously sterilised in a sterile container. Fill in to sterile glass ampoules, 10 ml/ampoule under aseptic conditions, and seal each ampoule by fusion of the glass.

b) The following suspension was prepared:

| | |
|---|---|
| Compound of Example 1, micronised | 1.0g |
| Polysorbate 20 | 0.1% w/v |
| Water for Injections | to 10 ml |

The Polysorbate 20 was dispersed in the water for injections, followed by the compound of Example 1. This suspension was filled into sterile glass ampoules, 10ml/ampoule under aspetic conditions, and the ampoules sealed by fusion of the glass.

B. Aerosol Formulations

a)

| | |
|---|---|
| Compound of Example 1, micronised | 1.0 mg |
| Aerosol propellant | to 5.0 ml |

Suspend the micronised naphthoquinone in the aerosol propellant. Fill this suspension into preformed aerosol cannisters, 5 ml/cannister under pressure, through the valve orifice.

b)

| | |
|---|---|
| Compound of Example 1, micronised | 1.0 mg |
| Arlacel 85 | 0.1% w/v |
| Aerosol propellant | to 5 ml |

Disperse the Arlacel 85 in the aerosol propellant and then add compound of Example 1. Fill the suspension into preformed aerosol cannisters, 5ml/cannister under pressure, through the valve orifice.

C. Powder Inhalation

| | |
|---|---|
| Compound of Example 1, micronised | 1.0 mg |
| Lactose | 29.0 mg |

Triturate and blend the micronised naphthoquinone with the lactose. Fill the resulting powder blend into hard gelatin capsule shells, 30 mg per capsule.

D. Nasal Drops

| | |
|---|---|
| Compound of Example 1 | 100.0 mg |
| Methylhydroxybenzoate | 10.0 mg |
| Water for Injections | to 10.0 ml |

Disperse the naphthoquinone and the methylhydroxybenzoate in the Water for Injections. Fill this suspension into suitable dropper bottles, 10 ml/bottle, and close by securing the dropper nozzle and bottle cap.

BIOLOGICAL TEST RESULTS.

Example 11

Activity against Pneumocystis carinii

Test Compounds

A: 2-[trans-4-(4-chlorophenyl)cyclohexyl]-3-hydroxy-1,4-naphthoquinone
B: 2-[cis-4-(4-chlorophenyl)cyclohexyl]-3-hydroxy-1,4-naphthoquinone
C: 2-[4-(4-chlorophenyl)cyclohexyl]-3-chloro-1,4-naphthoquinone
D: 2-(4-t-butylcyclohexyl)-3-hydroxy-1,4-naphthoquinone
E: 2-(4-t-butylcyclohexylmethyl)-3-hydroxy-1,4-naphthoquinone

a) Prophylaxis

Groups of 10 rats were treated with dexamethasone to allow latent Pneumocystis carinii infection to develop. Tetracycline was also administered to protect against bacterial infections. Test compound A was administered, by gavage, from day 4 of the dexamethasone treatment, at a dose of 100 mg/kg/day. Two control groups of rats were treated with dexamethasone and tetracycline only. A further group of rats was given cotrimoxazole (trimethoprim + sulphamethoxazole, 50 + 250 mg/kg/day, orally) in place of the test compound.

At the end of the test period the animals were sacrificed and autopsies carried out. The lungs were removed and the right lung bisected. An imprint was made onto microscope slides and stained with toluidine blue. One half of the lung was placed in formalin, processed in paraffin blocks, sectioned and stained by the Gomori methanamine silver nitrate method.

After autopsy the extent of P.carinii pneumonitis was scored under coded study as none if no organism seen; 1+ if P.carinii cysts seen sparsely distributed with less than one per 25 high power field (h.p.f.); 2+ if

focal areas of P.carinii pneumonitis surrounded by 10 to 25 h.p.f. of normal lung and 3+ if lung diffusely and extensively involved with organisms in almost all h.p.f.s.

Results

| | No of Rats | Early deaths or cannibalisation | No of with P.carinii Pneumonitis | | | | No of rats with PCP/No of rats tested |
|---|---|---|---|---|---|---|---|
| | | | None | 1+ | 2+ | 3+ | |
| Test Compound A | 10 | 2* | 8 | 0 | 0 | 0 | 0/8 |
| Control (1) | 10 | 2 | 0 | 1 | 2 | 5 | 8/8 |
| Control (2) | 10 | 0 | 0 | 0 | 2 | 8 | 10/10 |
| TMP/SMZ | 10 | 0 | | | | | 0/10 |

* one early death, believed due to gavage, one cannibalisation.

b) Prophylaxis

A further series of tests was carried out using the same general method as described above. Test compound A was administered at various dose levels, by gavage and in the diet.

The results are shown in Table 2.

TABLE 2

Extent of *P. carinii* after prophylaxis: histopathology of lung sections (Gomori-Grocott stain)

| Group (Dose per kg/day) g=gavage, r=rations | No.of Rats Tested per Group | No.of Rats* Evaluated | No.with *P. carinii* Pneumonitis: None | 1+ | 2+ | 3+ | Total No. |
|---|---|---|---|---|---|---|---|
| CONTROL: no drug | 10 | 9 | 0 | 1 | 0 | 8 | 9/9 |
| A: 200 mg (r) | 10 | 9 | 9 | 0 | 0 | 0 | 0/9 |
| A: 100 mg (r) | 10 | 10 | 10 | 0 | 0 | 0 | 0/10 |
| A: 100 mg (g) | 10 | 9 | 8 | 1 | 0 | 0 | 1/9 |
| A: 100 mg (g) | 15 | 9 | 9 | 0 | 0 | 0 | 0/9 |
| A: 50 mg (g) | 10 | 9 | 7 | 0 | 1 | 1 | 2/9 |
| A: 25 mg (g) | 10 | 8 | 1 | 2 | 1 | 4 | 7/8 |
| A: 10 mg (g) | 10 | 10 | 1 | 1 | 0 | 8 | 9/10 |
| TMP/SMZ: 50/250 mg (r) | 10 | 10 | 10 | 0 | 0 | 0 | 0/10 |

* - Excludes accidental deaths (gavage) and cannibalised rats.

c) Treatment

Groups of 10 rats were treated with dexamethasone and tetracycline for 4-6 weeks, as described in experiment (a) above. Three groups of rats were treated with Test compound A beginning after 4 weeks of immunosuppression, when Pneumocystis carinii pneumonia (PCP) had developed. Another group of rats in a parallel study was treated with Test compound A after 6 weeks of immunosuppression, when PCP infection was at an advanced stage. The results are shown in Table 3.

TABLE 3

Extent of *P. carinii* after prophylaxis: histopathology of lung section (Gomori-Grocott stain)

| Group (Dose per kg/day) g=gavage, r=rations | No.of Rats Tested per Group | No.of Rats Evaluated | No.with *P. carinii* Pneumonitis: None | 1+ | 2+ | 3+ | Total No. |
|---|---|---|---|---|---|---|---|
| (a) A: 100 mg (g) X3 wk | 10 | 8 | 8 | 0 | 0 | 0 | 0/8 |
| (a) A: 50 mg (g) X3 wk | 10 | 9 | 2 | 0 | 3 | 4 | 7/9 |
| (a) A: 25 mg (g) X3 wk | 10 | 8 | 1 | 0 | 1 | 6 | 7/8 |
| CONTROL: no drug* | 10 | 10 | 0 | 1 | 3 | 6 | 10/10 |
| CONTROL: no drug | 10 | 9 | 0 | 1 | 0 | 8 | 9/9 |
| TMP/SMZ: 50/250 mg (r) X3 wk | 10 | 10 | 8 | 1 | 1 | 0 | 2/10 |
| (b) A: 100 mg X 2 wk | 5 | 5 | 4 | 1 | 0 | 0 | 1/5 |
| CONTROL: no drug | 5 | 5 | 0 | 1 | 3 | 1 | 5/5 |

* 5/10 rats sacrificed at 4 weeks of immunosuppression the time when therapeutic drugs were started.

Dexamethasone and tetracycline continued throughout experiment in all animals.

a Treatment with test compound started after 4 weeks of dexamethasone

b Treatment with test compound started after 6 weeks of dexamethasone.

d) Treatment

Groups of 15 rats were treated with dexamethasone and tetracycline for 4 weeks, as described in experiment (a) above. Test compounds (A) - (E) were administered orally by stomach tube from the beginning of week 5 to the end of week 7.

In parallel with each test compound, Celacol was administered to one group of rats as a control. The results are given in Table 4.

TABLE 4

| Test Compound | GROUP (Dose/kg/day) | SCORE 0 | 1 | 2 | 3 | 4 | NO. INFECTED/ NO. EXAMINED | % INFECTED |
|---|---|---|---|---|---|---|---|---|
| | Celacol | 1 | 0 | 1 | 2 | 6 | 9/10 | 90 |
| A | 50mg/kg | 1 | 3 | 3 | 5 | 0 | 11/12 | 92 |
| | 75mg/kg | 2 | 5 | 2 | 1 | 2 | 10/12 | 83 |
| | 100mg/kg | 4 | 7 | 1 | 1 | 0 | 9/13 | 69 |
| | Celacol | 0 | 8 | 7 | 0 | 0 | 12/15 | 100 |
| A | 25mg/kg | 3 | 7 | 4 | 1 | 0 | 12/15 | 80 |
| | 50mg/kg | 1 | 6 | 4 | 2 | 0 | 12/13 | 92 |
| | 100mg/kg | 4 | 6 | 2 | 0 | 0 | 8/12 | 67 |
| | Celacol | 0 | 8 | 7 | 0 | 0 | 15/15 | 100 |
| B | 25mg/kg | 1 | 8 | 5 | 1 | 0 | 14/15 | 93 |
| | 50mg/kg | 3 | 6 | 4 | 0 | 2 | 12/15 | 80 |
| | 100mg/kg | 2 | 6 | 2 | 4 | 0 | 12/14 | 86 |

| | | | | | | | | |
|---|---|---|---|---|---|---|---|---|
| | Celacol | 0 | 8 | 7 | 0 | 0 | 15/15 | 100 |
| C | 25mg/kg | 0 | 5 | 6 | 3 | 0 | 14/14 | 100 |
| | 50mg/kg | 0 | 8 | 5 | 2 | 0 | 15/15 | 100 |
| | 100mg/kg | 4 | 6 | 4 | 1 | 0 | 11/15 | 73 |
| | Celacol | 0 | 8 | 2 | 3 | 2 | 15/15 | 100 |
| D | 25mg/kg | 0 | 7 | 7 | 1 | 0 | 15/15 | 100 |
| | 50mg/kg | 1 | 8 | 4 | 1 | 0 | 13/14 | 93 |
| | 100mg/kg | 3 | 8 | 3 | 0 | 1 | 12/15 | 80 |
| | Celacol | 0 | 8 | 2 | 3 | 2 | 15/15 | 100 |
| E | 25mg/kg | 1 | 3 | 2 | 4 | 4 | 13/14 | 93 |
| | 50mg/kg | 0 | 2 | 6 | 4 | 2 | 14/14 | 100 |
| | 100mg/kg | 1 | 4 | 2 | 6 | 0 | 12/13 | 92 |

## Claims

**1.** The use of a naphthoquinone of general formula (II)

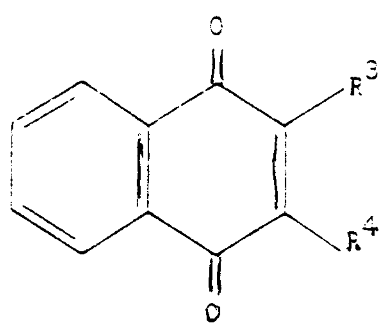

(II)

wherein

$R^3$ is $C_{1-35}$ non-aromatic hydrocarbon residue optionally substituted by one or more substituents, selected from halo, $C_{1-6}$alkoxy, hydroxy, phenyl, phenyl-$C_{1-6}$alkoxy and phenyl-$C_{1-6}$alkyl, each such

phenyl group or moiety being optionally substituted by one or more groups selected from $C_{1-6}$alkoxy, $C_{1-6}$alkyl, $C_{1-6}$alkoxy-$C_{1-6}$alkyl, hydroxy, halogen, halo-$C_{1-6}$alkyl, amino, and mono- or di-$C_{1-4}$alkylamino; and

$R^4$ is hydroxy; halogen;

a group $OCOR^5$, wherein $R^5$ is a $C_{1-10}$alkyl group, a $C_{3-10}$ cycloalkyl group, a $C_{1-10}$alkoxy group, or a phenyl or naphthyl group, each such $R^5$ group being optionally substituted e.g. by amino, mono- or di-$C_{1-4}$alkylamino, carboxy or hydroxy;

a group $OR^6$ or $SR^6$, wherein $R^6$ is an optionally substituted $C_{1-10}$alkyl, $C_{3-10}$cycloalkyl, phenyl or naphthyl group as defined for $R^5$; or

a group $NR^7R^8$, wherein $R^7$ and $R^8$ each independently represent hydrogen or $C_{1-4}$alkyl, or the group $NR^7R^8$ represents a 5-7 membered saturated heterocyclic ring, which may optionally contain a further heteroatom selected from nitrogen oxygen or sulphur;

and physiologically acceptable salts and other physiologically functional derivatives thereof for the manufacture of a medicament for the treatment and/or prophylaxis of Pneumocystis carinii infections in animals.

2. Use according to claim 1 wherein the naphthoquinone is selected from:-
2-(4-t-(butylcyclohexyl)-3-hydroxy-1,4-naphthoquinone
2-(4-t-butylcyclohexylmethyl)-3-hydroxy-1,4-naphthoquinone
2-[4-(4-chlorophenyl)cyclohexyl]-3-chloro-1,4-naphthoquinone
and physiologically acceptable salts and physiologically functional derivatives thereof.

3. Use according to claim 1 of 2-[4-(4-chlorophenyl)cyclohexyl]-3-hydroxy-1,4-naphthoquinone or a physiologically acceptable salt or other physiologically functional derivative thereof for the manufacture of a medicament for the treatment and/or prophylaxis of Pneumocystis carinii infections in mammals.

4. Use according to claim 3 wherein the compound is in the form of the trans isomer or a mixture of cis and trans isomers containing at least 95% of the trans isomer.

5. A pharmaceutical formulation which comprises a naphthoquinone as defined in any of claims 1 to 4 together with a pharmaceutically acceptable carrier therefor, adapted for nasal administration.

6. A pharmaceutical formulation which comprises a naphthoquinone as defined in any of claims 1 to 4 together with a pharmaceutically acceptable carrier therefor, adapted for pulmonary administration.

## Patentansprüche

1. Verwendung eines Naphthochinons der allgemeinen Formel (II)

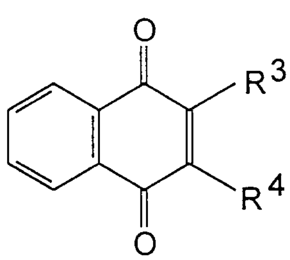

(II)

worin

$R^3$ ein nicht-aromatischer $C_{1-35}$-Kohlenwasserstoffrest ist, der wahlweise mit einem oder mehreren Substituenten, ausgewählt aus Halo, $C_{1-6}$-Alkoxy, Hydroxy, Phenyl, Phenyl-$C_{1-6}$-alkoxy und Phenyl-$C_{1-6}$-alkyl, substituiert ist, wobei jede solche Phenylgruppe oder -einheit wahlweise substituiert ist mit einer oder mehreren Gruppen, ausgewählt aus $C_{1-6}$-Alkoxy, $C_{1-6}$-Alkyl, $C_{1-6}$-Alkoxy-$C_{1-6}$-alkyl, Hydroxy, Halogen, Halo-$C_{1-6}$-alkyl, Amino und Mono- oder Di-$C_{1-4}$-alkylamino; und

$R^4$ ist:

Hydroxy; Halogen; eine $OCOR^5$-Gruppe, worin $R^5$ eine $C_{1-10}$-Alkylgruppe, eine $C_{3-10}$-Cycloalkylgruppe, eine $C_{1-10}$-Alkoxygruppe oder eine Phenyl- oder Naphthylgruppe ist, wobei jede solche $R^5$-Gruppe

wahlweise z.B. durch Amino, Mono- oder Di-$C_{1-4}$-alkylamino, Carboxy oder Hydroxy substituiert ist; eine $OR^6$- oder $SR^6$-Gruppe, worin $R^6$ eine wahlweise substituierte $C_{1-10}$-Alkyl-, $C_{3-10}$-Cycloalkyl-,Phenyl- oder Naphthylgruppe, wie für $R^5$ definiert, ist; oder eine $NR^7R^8$-Gruppe, worin $R^7$ und $R^8$ jeweils unabhängig Wasserstoff oder $C_{1-4}$-Alkyl darstellen oder die Gruppe $NR^7R^8$ einen 5- bis 7-gliedrigen gesättigten, heterocyclischen Ring darstellt, der wahlweise ein weiteres Heteroatom, ausgewählt aus Stickstoff, Sauerstoff oder Schwefel, enthalten kann;

und physiologisch annehmbaren Salzen und anderen physiologisch funktionellen Derivaten davon zur Herstellung eines Medikaments zur Behandlung und/oder Prophylaxe von Pneumocystis carinii-Infektionen bei Mensch und Tier.

2. Verwendung gemäss Anspruch 1, dadurch **gekennzeichnet,** dass das Naphthochinon ausgewählt ist aus:
2-(4-t-Butylcyclohexyl)-3-hydroxy-1,4-naphthochinon,
2-(4-t-Butylcyclohexylmethyl)-3-hydroxy-1,4-naphthochinon,
2-[4-(4-Chlorphenyl)cyclohexyl]-3-chlor-1,4-naphthochinon und physiologisch annehmbaren Salzen und physiologisch funktionellen Derivaten davon.

3. Verwendung gemäss Anspruch 1 von 2-[4-(4-Chlorphenyl)cyclohexyl]-3-hydroxy-1,4-naphthochinon oder eines physiologisch annehmbaren Salzes oder eines anderen physiologisch funktionellen Derivats davon zur Herstellung eines Medikaments zur Behandlung und/oder Prophylaxe von Pneumocystis carinii-Infektionen bei Säugern.

4. Verwendung gemäss Anspruch 3, dadurch **gekennzeichnet,** dass die Verbindung in der Form des trans-Isomers oder einer Mischung von cis- und trans-Isomeren, die mindestens 95 % trans-Isomer enthält, vorliegt.

5. Pharmazeutische Formulierung, umfassend ein Naphthochinon gemäss einem der Ansprüche 1 bis 4 zusammen mit einem pharmazeutisch annehmbaren Träger dafür, angepasst zur nasalen Verabreichung.

6. Pharmazeutische Formulierung, umfassend ein Naphthochinon gemäss einem der Ansprüche 1 bis 4 zusammen mit einem pharmazeutisch annehmbaren Träger dafür, angepasst zur pulmonalen Verabreichung.

## Revendications

1. Utilisation d'une naphtoquinone de formule générale (II) :

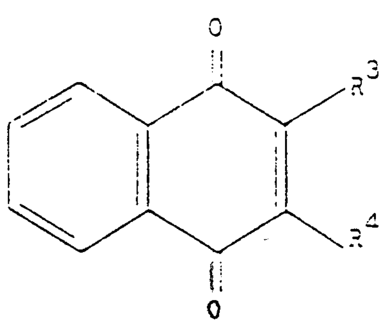

(II)

où

$R^3$ est un résidu hydrocarboné non aromatique en $C_{1-35}$ facultativement substitué en un ou plusieurs substituants choisis parmi halo, alcoxy en $C_{1-6}$, hydroxy, phényle, phénylalcoxy en $C_{1-6}$ et phénylalcoyle en $C_{1-6}$, chaque radical ou partie phényle étant facultativement substitué par un ou plusieurs radicaux choisis parmi alcoxy en $C_{1-6}$, alcoyle en $C_{1-6}$, (alcoxy en $C_{1-6}$)alcoyle en $C_{1-6}$, hydroxy, halogène, haloalcoyle en $C_{1-6}$, amino, et mono- ou di(alcoyle en $C_{1-4}$)amino, et

$R^4$ est hydroxy; halogène;

un radical $OCOR^5$, où $R^5$ est un radical alcoyle en $C_{1-10}$, un radical cycloalcoyle en $C_{3-10}$, un radical alcoxy en $C_{1-10}$ ou un radical phényle ou naphtyle, chaque radical $R^5$ étant facultativement substitué, par exemple, par amino, mono- ou di(alcoyle en $C_{1-4}$)amino, carboxy ou hydroxy;

un radical $OR^6$ ou $SR^6$, où $R^6$ est un radical alcoyle en $C_{1-10}$, cycloalcoyle en $C_{3-10}$, phényle ou naphtyle facultativement substitué comme défini pour $R^5$, ou

un radical $NR^7R^8$, où $R^7$ et $R^8$ représentent chacun indépendamment hydrogène ou alcoyle en $C_{1-4}$ ou le radical $NR^7R^8$ représente un cycle hétérocyclique saturé de 5-7 membres, qui peut facultativement contenir un autre hétéroatome choisi parmi azote, oxygène ou soufre;

et les sels physiologiquement acceptables et autres dérivés physiologiquement fonctionnels de ceux-ci pour la fabrication d'un médicament pour le traitement et/ou la prophylaxie d'infections par Pneumocystis carinii chez les animaux.

**2.** Utilisation suivant la revendication 1, où la naphtoquinone est choisie parmi :
2-(4-t-butylcyclohexyl)-3-hydroxy-1,4-naphtoquinone,
2-(4-t-butylcyclohexylméthyl)-3-hydroxy-1,4-naphtoquinone,
2-[4-(4-chlorophényl)cyclohexyl]-3-chloro-1,4-naphtoquinone,
et les sels physiologiquement acceptables et dérivés physiologiquement fonctionnels de ceux-ci.

**3.** Utilisation suivant la revendication 1 de la 2-[4-(4-chlorophényl)cyclohexyl]-3-hydroxy-1,4-naphtoquinone ou un sel physiologiquement acceptable ou autre dérivé physiologiquement fonctionnel de celle-ci, pour la fabrication d'un médicament pour le traitement et/ou la prophylaxie d'infections par Pneumocystis carinii chez les mammifères.

**4.** Utilisation suivant la revendication 3, où le composé est sous la forme de l'isomère trans ou d'un mélange des isomères cis et trans, contenant au moins 95% de l'isomère trans.

**5.** Formulation pharmaceutique qui comprend une naphtoquinone comme défini dans l'une quelconque des revendications 1 à 4, avec un excipient pharmaceutiquement acceptable de celle-ci, appropriée pour une administration nasale.

**6.** Formulation pharmaceutique qui comprend une naphtoquinone comme défini dans l'une quelconque des revendications 1 à 4, avec un excipient pharmaceutiquement acceptable de celle-ci, appropriée pour une administration pulmonaire.